# EUROPEAN PATENT SPECIFICATION

(11) **EP 1 348 428 B1**
(45) Date of publication and mention of the grant of the patent: **14.01.2009**
(21) Application number: 01998330.3
(22) Date of filing: 29.11.2001
(51) Int. Cl.: A61K 9/72, A61K 9/14, A61K 47/10, A61K 38/00, A61K 31/00

(54) **POWDERY PREPARATIONS AND PROCESS FOR PRODUCING THE SAME**
PULVERZUBEREITUNGEN UND VERFAHREN ZU IHRER HERSTELLUNG
PREPARATIONS PULVERULENTES ET SON PROCEDE DE PRODUCTION

(30) Priority: 29.11.2000 JP 2000362704; 26.03.2001 JP 2001088337; 29.11.2001 JP 2001364325
(43) Date of publication of application: 01.10.2003
(73) Proprietor: Itoham Foods Inc., Kobe-shi, Hyogo 657-0037 (JP)
(72) Inventor: ONOUE, Satomi, c/o Central Res. Inst.,, Ibaraki 302-0104 (JP); ENDO, Kousuke, c/o Central Res. Inst.,, Ibaraki 302-0104 (JP); KASHIMOTO, Kazuhisa, c/o Central Res. Inst.,, Ibaraki 302-0104 (JP)
(74) Representative: Woods, Geoffrey Corlett
(86) International application number: PCT/JP2001/010445
(87) International publication number: WO 2002/043703

(56) References cited:
- EP-A- 0 497 439
- EP-A- 1 238 661
- WO-A-94/17822
- WO-A-95/00127
- WO-A-95/24183
- WO-A1-94/17784
- JP-A- 8 301 762
- JP-A- 8 337 532
- JP-A- 11 171 760
- US-A- 5 898 028
- DATABASE WPI Section Ch, Week 200129 Derwent Publications Ltd., London, GB; Class B07, AN 2001-281802 XP002337949 & WO 01/26630 A1 (KAKEN PHARM CO LTD) 19 April 2001 (2001-04-19)
- TEE S K ET AL: "THE USE OF DIFFERENT SUGARS AS FINE AND COARSE CARRIERS FOR AEROSOLISED SALBUTAMOL SULPHATE" INTERNATIONAL JOURNAL OF PHARMACEUTICS, AMSTERDAM, NL, vol. 208, no. 1/2, 4 November 2000 (2000-11-04), pages 111-123, XP001031578 ISSN: 0378-5173
- MICHAUD J ET AL: "Erythritol: A new multipurpose excipient" PHARMACEUTICAL TECHNOLOGY EUROPE 2003 UNITED KINGDOM, vol. 15, no. 10, 2003, pages 69-72, XP009051288 ISSN: 0164-6826
- ENDO K ET AL: "Erythritol-based dry powder of glucagon for pulmonary administration" INTERNATIONAL JOURNAL OF PHARMACEUTICS, AMSTERDAM, NL, vol. 290, no. 1-2, 16 February 2005 (2005-02-16), pages 63-71, XP004967391 ISSN: 0378-5173

## Description

### Technical Field

The present invention relates to powder formulations utilizing carriers, and more particularly to powder formulations that can be used as a good inhalant formulations of peptides or proteins, which are often difficult to be made into formulations. In addition, the resulting formulations can be easily handled pharmaceutically and can retain a constant medicament content because of improved dispersibility and flowability.

### Background of the Invention

Inhalation therapy is a method utilizing transrespiratory medicaments applied to the diagnosis of diseases, transbronchial systemic drug administration, disease prevention, transbronchial immune desensitising therapy, and other area as well as the therapy of airways diseases. In any of these cases, however, methods for determining the applications of this therapy have not been well examined, and development of inhalants dealing with such needs has been awaited. When applied as targeting therapy, a standard method for selecting an inhalant should be considered based not only on the efficacy on the disease, but also based on the method of medicament particles generated, the site reached by medicament particles, and the correlation of fundamental properties between these factors and medicaments. Currently, this form of medicament is extensively used in aerosol inhalation therapy. For example, WO 95/24183 and US 5,898,028 relate to methods and compositions for pulmonary delivery of insulin. Other examples of agents that have actually been used are bronchodilators, agents for solubilizing mucous membranes, antibiotics, antiallergic agents, steroid medicaments, vaccines, and physiologic saline. When they are clinically applied, the site of action, mechanism of action, composition, directions for use, or the like of inhalants are considered to be important factors.

Recently, dry powder inhalers (DPIs) have begun to draw attention in the treatment of bronchial asthma and chronic obstructive lung disease. Suitable dry powders and processes for producing them are described in, for example, WO 01/26630 and EP-A-1238661. Inhalants include the aforementioned aerosols using metered dose inhalers (MDIs) and agents using nebulizers. These agents possess various beneficial properties. For example, the surface area of a lung, which is a target site of these agents, is almost as large as that of the small intestine, and there is no first-pass effect after absorption. Examples of acknowledged properties of general inhalants are 1) rapid development of medicament effects, 2) gradual reduction of side effects, 3) sufficiency of small dosage, and 4) avoidance of the first-pass effect.

As described above, MDIs have been extensively used and have been still intensively researched, however, they have yet to utilize chlorofluorocarbons as a propellant Thus, environmental problems are pointed out. Although development of alternatives to chlorofluorocarbons has been attempted in order to solve this problem, development of DPIs has been more actively attempted because DPIs do require chlorofluorocarbons and provides excellent portability. Nebulizers are also considered to be inadequate for unstable medicaments since medicaments have to be stored in the device in a dissolved state. Accordingly, DPIs are useful also in view of long-term stability, since medicaments can be stably stored therein.

DPIs are classified into capsule, lister, reservoir, or other types depending on the type of packaging. At the time of administration using a DPI, pores for inhalation are usually provided on capsules, etc. by operating the device in order to take out the medicaments encapsulated therein, medicaments are released by inhalation operation, and they are then administered to the bronchi, lungs, or other locations.

Regarding DPIs, there is a close correlation between the size and deposition of medicament particles on airways inhaled by a patient (Pharmacia (1997), vol. 33, No. 6, 98-102). Also acknowledged is the aerodynamic correlation, i.e., the sufficient size of medicament particles to deposit in the bronchi and lungs. It is generally known that the aerodynamically optimal size of medicament particles that can reach the site in the bronchi and the air vesicle is about 1 µm to 6 µm (Int. J. Pharm. (1994) 101,1-13).

Several µm-sized particles reach the air vesicle, where they are efficiently absorbed through the lung mucosa and then migrate to the blood. Accordingly, this particle size is particularly important when actions on the whole body are intended. As the sizes of particles are reduced, however, particles are more likely to cohere due to various factors. This deteriorates the fluidity of powders and generates concerns regarding lowered loading accuracy and handleability at the time of production. Further, it could be presumed that particles adhere in the device or capsule at the time of administration.

In the dosage form design for powder formulations, formulations utilizing carriers are often used these days as described in, for example, Int.J.Pharm. (2000) 208(1), p.111-123 and JP-A-8/337532. That is, medicament-containing compositions are finely ground with a jet mill or many other conventional techniques, and mixed with carriers to form a group of particles having relatively large particle sizes. This improves, for example, the fluidity and the handleability at the time of loading into capsules. A specific example of a well-known method is the one in which coarse particles such as lactose, which is a carrier, are mixed with finely ground medicaments. This can be achieved because the adsorption of finely ground medicaments on the surface of lactose through intermolecular interactions can attenuate the cohesion of these medicaments and can increase the particle size as a whole. This enhances the fluidity of formulations.

Other methods include medicament granulation and surface modification. For example, improved fluidity through medicament granulation is proposed in "Soft-pellet medicaments and processes for the formulation thereof" (WO 99/27911).

Unlike soft-pellet medicaments, a dosage form utilizing a carrier requires uniform medicament distribution from the viewpoint of the chemical properties of the formulations. There are many medicaments in which a phenomenon referred to as "self-aggregation" is observed, regardless of whether they are natural or synthetic products. Thus, it is often difficult to uniformly mix medicaments with carriers.

In addition, peptides and proteins generally have very high hygroscopic properties, and many of peptides and proteins deliquesce when they are allowed to stand for a long period of time, or even for a short period of time depending on the type of compound. It is easy to presume that moisture absorption significantly affects the properties of formulations. This also significantly affects production efficiency when producing formulations. Therefore, a very important factor is how to finely grind peptides and proteins effectively and maintain them as dried powders.

As described above, peptides and proteins have many factors that make them unsuitable for the DPI. However, these peptides and proteins contain many very useful medicaments and can often provide sufficient pharmacological effects with a very small dosage. From the viewpoint of medical economics, therefore, a useful dosage form design with which peptides and proteins are made into transpulmonary medicaments or inhalants has been awaited.

In contrast, carriers, which are actually provided in the medical field and commonly used in the dosage form design, are likely to absorb moisture in general. The dissociation rate between medicaments and carriers is low due to high cohesion with medicaments, and the rate of migration into the lung is lowered due to the deposition of formulations in the device. In this case, formulations are most likely to be deposited in the throat, and some medicaments have very poor tastes. When medicaments have irritant actions on the throat mucosa, they impose a significant burden on patients. Further, low bioavailability results in increased amounts of formulations to be administered. This could further impose economic burdens on patients. When compounds such as lactose are used as carriers, some medicaments have difficulties in terms of generating good aerosols, have very low rate of reaching the lung, and cannot avoid deposition on the throat. Thus, this development of formulations was still insufficient to solve the aforementioned problems. Therefore, a dosage form design that can solve these various problems has been desired from the viewpoint of medical economics.

An object of the present invention is to develop a dosage form design in which self-associating medicaments can be uniformly mixed with carriers and a medicament form in which a very small amount of a medicament can be uniformly mixed with carriers. It is another object to impart a novel form of administration that is capable of maintaining the dried state without any detriment to various medicaments, which are unstable in a solution but very useful. It is also an important object to maintain good production efficiency at the time of production with some contrivances regarding the dosage form.

A further object of the present invention is to develop a medicament form in which dissociation between carriers and medicament particles is facilitated and the rate of migration into mucosal tissues of, for example, bronchi or lungs can be improved. This can prevent the medicament particles from being accumulated in mucosal tissues and inhibit local irritation.

### Disclosure of the Invention

We have conducted concentrated studies in order to attain the above objects. As a result, we have succeeded in obtaining very uniform formulations by mixing appropriate excipients and medicaments in a solution or aerodynamic mill, producing medicaments having targeted particle sizes using mills such as a jet mill, and mixing the resulting medicaments with carriers. This has led to the completion of the present invention. In addition, we have succeeded in remarkably improving solubility with the use of erythritol as an excipient. This dosage form can be also used for transpulmonary medicaments, inhalants, and powdery transnasal formulations.

Furthermore, we have succeeded in preventing strong binding between medicaments and carriers from occurring by incorporating the low-hygroscopic and low-cohesive compound erythritol as a carrier. Various further synergistic effects were obtained with the use of erythritol as an excipient.

Specifically, the present invention provides the following (1) to (16):
(1) A powder formulation for transpulmonary administration, inhalation or transnasal administration comprising a mixture of (a) fine particles containing powdery medicament and erythritol as an excipient and having average particle diameter of not more than 20 µm with (b) a carrier having a particle diameter of 10-200 µm wherein the carrier is erythritol.
(2) The powder formulation according to (1) above, wherein the medicament is capable of self-aggregation through intermolecular interactions.
(3) The powder formulation according to (1) above, wherein the medicament has hygroscopic and/or deliquescent properties.
(4) The powder formulation according to any one of (1) to (3) above, wherein the medicament is a peptide or a protein.
(5) The powder formulation according to any one of (1) to (4) above, wherein the medicament is encapsulated within a lipid layer.
(6) The powder formulation according to any one of (1) to (5) above, wherein the proportion of the powdery medicament to the excipient is in the range of 1:5000 to 10:1 by weight.
(7) The powder formulation according to any one of (1) to (6) above, wherein the proportion of fine particles to carriers is in the range of 1:100 to 10:1 by weight.
(8) A method for producing a powder formulation, comprising the steps of: dissolving or suspending a powdery medicament in a solution of erythritol as an excipient; spray-drying the resultant solution or suspension as such or disrupting a dried product obtained by lyophilizing the solution or suspension; thereby yielding fine particles having an average particle diameter of not more than 20 µm, and subsequently mixing the fine particles with a carrier having a particle diameter of 10-200 µm wherein the carrier is erythritol.
(9) The method according to (8), wherein the medicament is capable of self-aggregation through intermolecular interactions.
(10) A method for producing a powder formulation as defined in any one of (1) to (7), comprising the steps of:
   simultaneously grinding and mixing erythritol as an excipient and a powdery medicament using an aerodynamic grinder; thereby yielding fine particles having an average particle diameter of not more that 20µm, and subsequently mixing the fine particles with a carrier having a particle diameter of 10-200 µm wherein the carrier is erythritol.
(11) The method according to (10), wherein the medicament is capable of self-aggregation through intermolecular interactions.
(12) An inhaler comprising a powder formulation as defined in any one of (1) to (7).
(13) The powder formulation according to any one of (1) to (7), wherein the fine particles can be easily separated from the carriers after administration and can target sites such as the bronchi or air vesicles.

### Brief Description of Drawings

Fig. 1 shows the particle diameter distribution of fine particles containing only lactose before mixing with carriers.
Fig. 2 shows the particle diameter distribution of fine particles containing glucagon and lactose before mixing with carriers.
Fig. 3 shows the particle diameter distribution of carriers (Pharmatose 325 M) only.
Fig. 4 shows the particle diameter distribution of the complex after mixing fine particles with carriers (Pharmatose 325 M).
Fig. 5 shows the glucagon peak area of each sample determined by HPLC.
Fig. 6 shows the analyses results on a mixture of finely ground erythritol and lactose carrier obtained using a cascade impactor.
Fig. 7 shows the analyses results on a mixture of finely ground mannitol and lactose carrier obtained using a cascade impactor.
Fig. 8 shows the analyses results on a mixture of finely ground lactose and lactose carrier obtained using a cascade impactor.
Fig. 9 shows changes in blood glucose levels through transpulmonary administration of glucagon-DPI:
   (a) elevation of blood glucose level after administration (15 minutes) of glucagon-DPI, which is not encapsulated within liposome, compared to before administration;
   (b) elevation of blood glucose level after administration (15 minutes) of glucagon-DPI, which is encapsulated within liposome, compared to before administration; and
   (c) comparison of changes in blood glucose level between glucagon-DPI, which is not encapsulated within liposome, and glucagon-DPI, which is encapsulated within liposome.

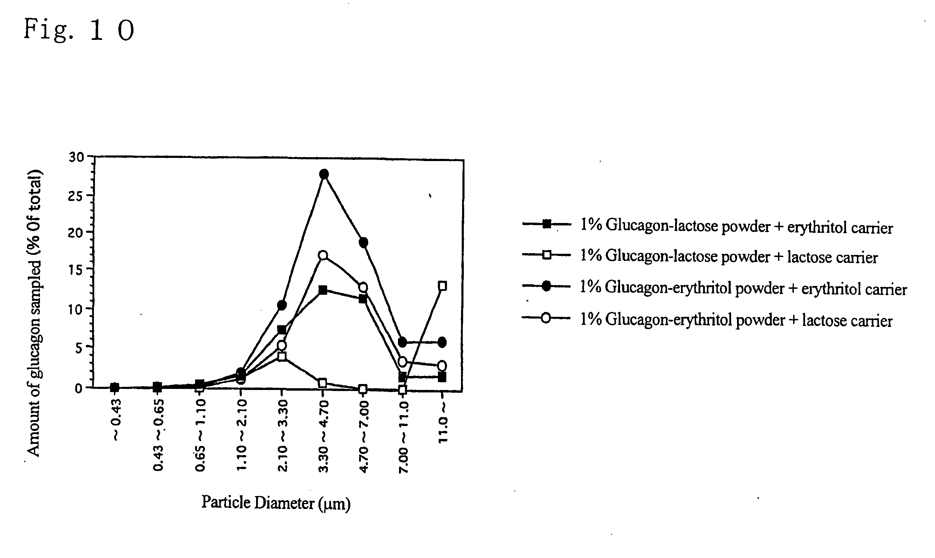

Fig. 10 shows the analyses results on glucagon formulations obtained using a cascade impactor.

### Description of Sequence Listings

SEQ ID NO: 1 represents a sequence of VIP derivative used in the present invention.

### Embodiments for carrying our the Invention

The present invention is hereafter described in more detail.

### [1] Medicaments

The medicaments used in the present invention are not particularly limited and include those that are used or will be used in the future as pharmaceuticals. Medicaments include those which can be obtained in a powdered state, and those which can be processed into powders by grinding or other processing. The forms, particle diameters, or other properties of powdery medicaments that can be used in the present invention are not particularly limited. In the case where medicaments have properties of self-aggregation through intermolecular interactions, moisture absorption, and deliquescence, the present invention can be particularly effectively used. Examples of medicaments having properties of self-aggregation through intermolecular interactions include glucagon (J. Biol. Chem. (1984) 259, 7031-7), FGF (Biochem. J. (1999) 341, 613-20), growth hormone (Biochemistry (1993) 32, 1555-62), endothelin (Pept. Res. (1992) 5, 97-101), calcitonin (Biochem. Biophys. Res. Commun. (1998) 245, 344-8), insulin (Int. J. Pharm. (1999) 191, 51-64), and glucagon-like peptide-1 (Pharmaceutical Research (1998) 15, 254-62). In the present invention, medicaments are preferably those in which uniform mixing for formulations is difficult due to very small dosages. The present invention can be applied to, but are not limited to, for example, a series of angiotensin converting enzyme inhibitors, LH-RH, ACTH, caerulein, CCK, salmon calcitonin, EGF, G-CSF, GM-CSF, oxitocin, neurotensin, ghrelin, PTH, PTHrP, somatostatin, VIP, PACAP, vasopressin, enkephalin, endorphin, dynorphin, substance P, β-NGF, TGF-β, erythropoietin, interferon-α, β, or γ, interleukin, tumor necrosis factors, GHRF, c-GRP, ANP, CRF, secretin, immunosuppressive agents such as cyclosporin, α-MSH, and a series of serine proteases such as thrombin and plasmin or derivatives containing their agonists and antagonists. The formulations according to the present invention can also be utilized in the direct administration in the treatment of lung diseases, for example, lung cancer or lung pneumothorax.

Even though peptides and proteins are very expensive, it is demonstrated that they aggregate with each other under specific conditions such as a condition of high concentration. For example, the aforementioned calcitonin undergoes self-aggregation at concentrations of 50 mg/mL or higher. Hydrogen bonds, hydrophobic interactions, and other various interactions are observed in a group of peptide and protein compounds. The possibility of physicochemical denaturation such as aggregation or sedimentation at the time of production is pointed out. According to the present invention, however, these problems can be prevented beforehand.

The formulation of the present invention is also effective in, for example, steroids or β-stimulants, the systemic actions of which are not preferable, and medicaments, the local actions in the bronchi of which are preferable. The formulation can be used in many medicaments, the use of which is desirable in domiciliary treatment, as a simple administration method avoiding the first-pass effect and causing no pain. The formulation can also be widely used for non-peptide and non-protein medicaments. Specific examples include anti-inflammatory steroids or nonsteroidal antiphlogistics, analgesic-antiphlogistics, sedatives, therapeutic agents for depression, antitussive expectorants, antihistamic drugs, antiallergic drugs, antiemetics, sleep inducers, vitamin formulations, sex steroid hormones, antitumor drugs, antiarrhythmic drugs, hypertension drugs, antianxiety drugs, psychotropic drugs, antiulcer drugs, cardiac restoratives, analgesics, bronchodilators, obesity drugs, antiplatelet coagulants, antidiabetic drugs, muscle relaxants, migraine drugs, and antirheumatics. These may be used solely or in combinations of two or more.

### [2] Excipients

In the present invention, it is advantageous to use medicaments in combination with excipients, thereby forming fine medicament-containing particles. This is because uniform mixing of carriers with fine particles is difficult when producing formulations with a very low medicament content. It is also possible to inhibit the aggregation caused by the interaction between medicaments, although this is not feasible when formulations contain relatively large amount of medicaments and have low association.

The term "fine particles" used herein refers to finely ground medicaments or finely ground mixture of medicaments and excipients.

In general, excipients are added for the purposes of loading, diluting, filling, or shaping solid formulations such as powders or tablets, and they significantly affect the release properties of active components. Thus, selection or modification thereof should be made carefully. In the present invention, excipients are effective to enhance the solubility of medicaments and/or to lower the ability of self-aggregation. Accordingly, preferable excipients easily dissolve in water. However, excipients that significantly absorb moisture are not preferable in terms of the properties of the present formulations.

Since erythritol is used as an excipient in the powder formulations according to the present invention, fine medicament-containing particles can be more easily dissociated from carriers. This is considered to be a useful effect that can be attained by improving the properties of whole fine particles through the low cohesiveness of erythritol.

In the formulations according to the present invention, the proportion of the powdery medicaments to the excipients is preferably in the range of 1:5000 to 10:1 by weight. If the amount of medicaments exceeds the upper limit of this range, the content uniformity could be disturbed. If the excipients are increased, some types of medicaments could lose their pharmacological activities.

In contrast, a formulation technique in which an enhancer is added or a method of preparing formulations in which medicaments are encapsulated within a lipid layer have recently become well known as techniques for enhancing the rate of absorption in DPI. Examples of known enhancers are organic acids such as citric acid and capric acid, enzyme inhibitors such as bacitracin, and NO generators (Drug Delivery System (2001) 16, 297; Drug Delivery System (2001) 16, 299). As substrates for liposome, high molecular weight polymers such as chitosan are well known. The use of these substances is mainly aimed at improving the membrane permeability of medicaments, and the rate of formulations reaching the target tissue (lungs or bronchi) is not always enhanced. Even though the rate of absorption is remarkably enhanced, clinical application is not always possible unless the medicament reaches the target tissue. Accordingly, the application of this method of preparing formulations to a dosage form design with some contrivances for some purposes can further yield beneficial formulation properties.

### [3] Carrier

In the present invention, carriers are used to prevent medicaments from associating with each other before the administration of powder formulations. At the time of administration, carriers are used to efficiently dissociate medicaments and enhance their absorption efficiency at the time of inhalation operation using an inhaler. When carriers are used in designing DPI formulation, it is preferable that medicaments are definitely released from capsules or devices and separated from the surfaces of carriers with a high probability. Thus, the dosage form design should be made with thorough consideration. In the use of carriers, fluidity of formulations, prevention of medicament association, whether or not the dosage can be increased or decreased, and the like are important. Accordingly, major concerns regarding standards for selecting carriers are ease and operability at the time of handling, not to mention toxicity and physicochemical stability.

In the present invention, the carrier is erythritol. Erythritol can be very easily dissociated from medicaments. This can yield a high rate of migration to the target tissue and can reduce accumulation in the throat. Thus, burdens on users imposed by tastes of medicaments can be reduced. With this dosage form design, however, carriers remain in the throat. In such a case, a foreign-body sensation caused thereby is an issue of concern. Erythritol has very high solubility, and this effect can be utilized for intraorally disintegrating agents (JP Patent Publication (Unexamined Application) No. Hei-09-316006). This can prevent or very rapidly eliminate the discomfort of carriers remaining in the throat. In order to further enhance the rate of formulations for migrating to the tissue, the medicament content of the formulations can be reduced compared to those in other forms of formulations. This can induce lowering of the costs necessitated in production, and reduce economic burdens on patients. Sweetness of the present compound is the highest among many sugar alcohols, i.e., when the sweetness of sucrose is determined to be 100, that of erythritol is 75. This can improve the tastes of formulations deposited in the throat. This taste improvement can be further achieved not only by sweetness of erythritol but also by the bitterness masking effect recognized in the present compound (JP Patent Publication (Unexamined Application) No. Hei-10-306038). In addition, the present compound, which does not cause the Maillard reaction, is considered to prevent the formulations from undergoing deteriorations such as those involving coloring and to lessen the spoilage of formulations' appearances. Inhalants are often developed for domiciliary treatment as opposed to intramural treatment. Because of such conditions, application thereof to therapeutic agents for diabetes is considered. The energy value of erythritol (kcal/100 g) is almost zero, and influence on the blood glucose level is very small. Accordingly, erythritol can be continuously used in the treatment of diabetes for a long period of time. When administering inhalants, patients sometimes become anxious about whether or not they have actually inhaled formulations. When erythritol is inhaled, however, a cool sensation is clearly recognized. With this cool sensation, the inhalation of formulation can be confirmed.

Formulations using sugar alcohol as a carriers are considered to have much lower hygroscopic properties than those using lactose as a carrier and to be able to be used clinically regardless of the weather.

The powder formulations according to the present invention are administered using inhalers. Accordingly, carriers have aerodynamically acceptable particle sizes. Specifically, the particle sizes of carriers are in the range of 10 µm to 200 µm.

When only actions as carriers are intended in terms of dosage form design, it is known that enlargement of particle sizes is sufficient. It is also known that carriers remain in the throat or oral cavity if the particle sizes are enlarged. Accordingly, when carriers per se are biologically inert and prevention of carriers from reaching the lung is desired, sufficient particle sizes are at least 10 µm. When the optimal condition is further desired, crude materials are desirably selected in consideration of, for example, compatibility of the main component with the excipient mixed therewith. Unless there are major problems, materials of the carriers selected are preferably similar to those of the excipients.

According to the present invention, the proportion of fine particles to carriers is preferably in the range of 1:100 to 10:1. If the amount of fine particles exceeds the upper limit of this range, the content uniformity could be disturbed. If the carriers are increased, some types of medicaments could lose their pharmacological activities.

### [4] Steps of mixing and grinding medicaments and excipients

In the production of fine medicament-containing particles, medicaments or medicaments and excipients should be first finely ground. When medicaments and excipients are incorporated in the formulations, the production process comprises steps of mixing and grinding medicaments and excipients. Grinding is carried out as follows. For example, a solution or suspension of medicaments or medicaments and excipients is lyophilized. The resulting lyophilized product is then ground. Alternatively, medicaments or medicaments and excipients can be directly mixed and ground using an aerodynamic mill.

The method for producing fine particles according to the present invention is not particularly limited, and any conventional methods known to those skilled in the art can be used when suitable. Examples of such methods include a spray drying method in which a solution is converted into powder and a method using supercritical fluid. The former method is a conventional technique and the latter method is one of the relatively new concepts which have particularly begun to draw attention recently. Examples of methods for producing medicament particles with the use of supercritical fluid include the precipitation with compressed antisolvent (PCA) method, the rapid expansion of supercritical fluid solutions (RESS) method, and the gas antisolvent (GAS) method. Among these, the PCA method has begun to draw attention recently. In this method, medicaments are dissolved in dimethyl sulfoxide (DMSO) and sprayed in supercritical CO₂, thereby producing fine particulate powders. This method is also applicable to proteins. For example, Winters et al. produced fine particulate powders (15 µm) of lysozyme, trypsin, and insulin by the PCA method (J. Pharm. Sci. (1996) 85,586).

The method that is used to produce fine particles in the present invention can be suitably determined depending on, for example, types of medicaments and excipients or the final size of particles. Crystal conditions of compounds and properties of formulations such as cohesiveness and dispersibility are often correlated with each other. Accordingly, the latter treating method should be desirably selected in this step. It should be noted that, when erythritol is used, good ground mixtures can be obtained by any method because of the very high crystal orientation of the compounds.

In the present invention, medicaments or medicaments and excipients can be ground by a general dry grinding process. The use of an aerodynamic mill is particularly preferred. Specifically, a device, which efficiently grinds a small amount of medicaments or medicaments and excipients such as a mortar or ball mill, is commonly used in laboratories as a general dry mill. Examples of known ball mills include rolling ball mills, centrifugal ball mills, vibrating ball mills, and planetary ball mills. These mills can grind medicaments or medicaments and excipients based on the principles of abrasion, spinning, vibration, or impact. At industrial levels, devices such as medium-agitation-type mills, high-speed spinning abrading and impact mills, and jet mills are often used to efficiently grind a large amount of raw materials. High-speed spinning abrading mills include disc mills and roller mills. High-speed spinning impact mills include those milling by shear forces in addition to those milling by spinning impact such as cutter mills (knife mills), hammer mills (atomizers), spin mills, and screen mills. Many jet mills perform milling mainly by impact. Examples thereof include the most traditional particle-particle impact type, particle-impact plate impact type, and nozzle-suction type (blowing type) mills.

In this grinding step, medicaments or medicaments and excipients are finely ground to fine particles having an average particle size of not more than 20 µm. With this particle size, fine particles can be easily separated from carriers after the administration and can reach target sites such as the bronchi or air vesicles.

### [5] Step of mixing carriers and fine particles

The fine particles obtained in the mixing and grinding step were then mixed with carriers to form complexes that were stable until administration.

Carriers and fine particles can be mixed using a conventional mixer. The main types of mixers are batch types and continuous types. Batch type mixers are further classified into two types, i.e., a spinning type and a fixed type. Spinning types include horizontal cylindrical mixers, V-shape mixers, double conical mixers, and cubic mixers. Fixed types include screw (vertical, horizontal) mixers, rotating screw mixers, and ribbon (vertical, horizontal) mixers. Continuous types are also classified into two types, i.e., a spinning type and a fixed type. Spinning types include horizontal cylindrical mixers and horizontal conical mixers. Fixed types include screw (vertical, horizontal) mixers, ribbon (vertical, horizontal) mixers, and spinning disc mixers. In addition, uniformly mixed formulations can be produced by a mixing method utilizing aerodynamic mills such as medium-agitation-type mills, high-speed spinning abrading and impact mills, and jet mills, or by agitating in a nylon bag or a bag having properties in conformity therewith.

### [6] Inhalers

The powder formulations of the present invention obtained in the above step can be administered to a subject transmucosally through, for example, a transpulmonary or transnasal route. More specifically, when the formulations are administered through a transpulmonary route, any inhalers that are used in the art can be used for administration.

Examples of inhalers that can be used include, but are not limited to, devices for inhalation and transpulmonary administration and metered nebulizers such as Spinhaler, E-haler, FlowCaps, Jet-haler, Disc-haler, Rotor-haler, Inspir-Ease, and Inhalation Aid.

### EXAMPLES

The present invention is hereinafter described in more detail with reference to Examples, although it is not limited thereto.

### [Example 1] Production of excipient powders

Lactose, mannitol, and erythritol were finely ground under the following conditions.

### (Conditions for grinding)

| | |
|---|---|
| Equipment used: | A-O-Jet Mill (Seishin Enterprise Co., Ltd.) |
| Means for feeding materials: | Automatic feeder |
| Feeding air pressure: | 6.0 kg/cm² G |
| Grinding air pressure: | 6.5 kg/cm² G |
| Dust collection: | Collecting bag (polyethylene) |

Yields were as follows.

| | |
|---|---|
| Lactose: | 32.0% |
| Mannitol | 50.5% |
| Erythritol | 75.7% |

These results demonstrate that a sugar alcohol is better as an excipient of the present invention than lactose in terms of yield.

### [Example 2] Production of glucagon powders (a Reference Example)

| | |
|---|---|
| Equipment used: | Spiral Jet Mill 50 AS (Hosokawa Micron Corporation) |
| Feeding materials: | Means for feeding: Hand feed |
| Feeding air pressure: | 4.7 kg/cm²G |
| Diameter of injector nozzle: | 0.9 mm |
| Nozzle position: | Backmost |
| Grinding conditions: | Grinding air pressure: 4.5 kg/cm² G |
| Diameter of nozzle ring | 0.9 mm |
| Number of nozzles: | 4 |
| Angle of incidence from nozzle, | 30 degrees |
| Dust collection: | Collecting bag (polyethylene) , |

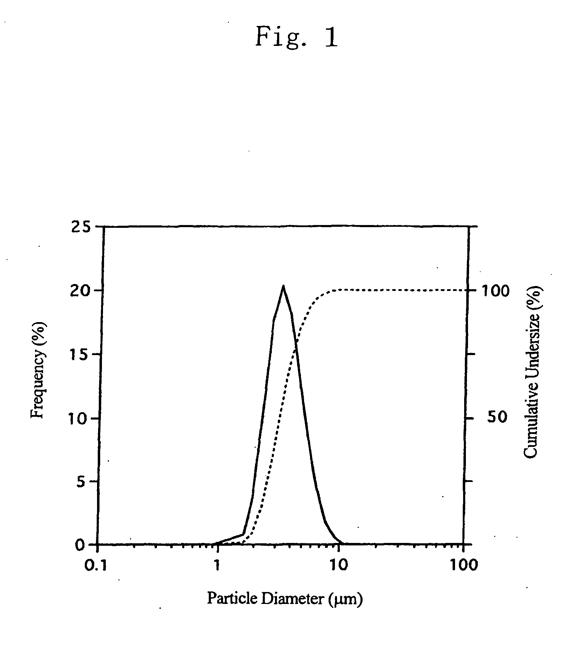

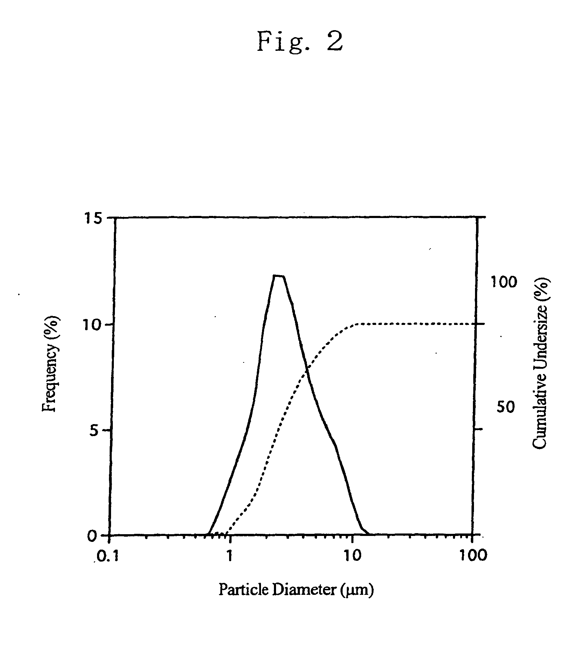

In accordance with the aforementioned method, about 10 g each of lactose listed in the Japanese Pharmacopoeia (Yoshida Pharmaceutical Co., Ltd.) and lyophilized 1% glucagon-lactose powder were ground. When treating a certain amount, i.e., 10 g, yield of each substance was almost the same as shown in Table 1, and they have almost same appearance based on visual inspection for their properties. Aggregation of glucagon, which had been an issue of concern, was seemingly solved according to visual inspection, and it was confirmed that glucagon was a powder that exhibited almost the same properties as lactose. Further, particle diameter distributions of both substances were evaluated using a laser diffraction/scattering-type particle diameter distribution analyzer (Nikkiso Co., Ltd.). As shown in Figs. 1 and 2, there were some differences in distribution ranges, although the peak was located at about 3 µm and substantially 100% of powders had particle diameter of not more than 10 µm, respectively.

**Table 1**

| (Grinding of lactose) | | (Grinding of 1% glucagon-lactose powders) | |
|---|---|---|---|
| Amount fed | 101.0 g | Amount fed | 10.0 g |
| Recovered powders | 96.8 g | Recovered powders | 9.4 g |
| Yield | 95.8% | Yield | 94.0% |

### [Example 3] Production of small amounts of glucagon powders

In the same manner as in Example 1,2 g of both lyophilized 1% glucagon-lactose powders and 1% glucagon-erythritol powders were ground, respectively. Yields are shown in Table 2. As is apparent from these results, when a small amount of powder is produced, many factors such as adsorption and moisture absorption, which lower yields, arise in a more visible manner. Thus, the yield of mixtures using erythritol became higher. Aggregation of glucagon, which had been an issue of concern, was seemingly solved based on the visual inspection, and it was confirmed that these powders exhibited almost same property as the fine particles of excipients produced in Example 1.

**Table 2**

| Material | Yield |
|---|---|
| 1 % glucagon-lactose powders | 61.2% |
| 1 % glucagon-erythritol powders | 75.5% |

### [Example 4] Production of buserelin acetate powders

Buserelin acetate (Itoham Foods Inc.), a mixture of buserelin acetate and lactose (570-fold diluted powder), a mixture of buserelin acetate and mannitol (570-fold diluted powder), and a mixture of buserelin acetate and erythritol (570-fold diluted powder) were finely ground in the same manner as in Example 1.

Yields were as follows.

| | |
|---|---|
| Buserelin acetate only | Unconfirmed due to deliquescence |
| Mixture of buserelin acetate and lactose | 27.0% |
| Mixture of buserelin acetate and mannitol | 41.7% |
| Mixture of buserelin acetate and erythritol | 71.7% |

As is apparent from these results, it is very difficult to grind peptides alone. Also, the effect of the addition of excipients such as lactose or erythritol was demonstrated. The mixture with lactose had very strong cohesiveness, and thus, recovery from a bag filter was somewhat difficult. In contrast, the mixture with mannitol or erythritol had very low cohesiveness, and their recovery rates were remarkably enhanced.

### [Example 5] Production of powders containing glucagon at a high level

Mixtures of glucagon and erythritol (3-fold, 5-fold, 10-fold, and 20-fold diluted powders) were finely ground using a jet mill as in Example 1 under the following conditions. Specifically, glucagon and erythritol were mixed at the ratio as shown in the table below, and they were ground into fine particles using a jet mill under the conditions as specified in Example 1. Thus, a mixture of finely ground glucagon and erythritol was recovered from a bag filter for powder collection. Recovery rates were about 30 to 40% in all cases. It was confirmed by quantitative HPLC analysis that the glucagon content in the powders was almost equal to the theoretical value. This method of preparing formulations can produce such high concentration peptide formulations.

**Table 3**

| Dilution of glucagon powder | Amount of glucagon | Amount of erythritol |
|---|---|---|
| 20-fold | 20 mg | 380 mg |
| 10-fold | 40 mg | 360 mg |
| 5-fold | 80 mg | 320 mg |
| 3-fold | 150 mg | 300 mg |
| 1-fold | 100 mg | 100 mg |

### [Example 6] Production of vasoactive intestinal peptide (VIP) derivative powders

A mixture of VIP and erythritol (400-fold diluted powders) was finely ground using a jet mill as in Example 1 under the following conditions. Specifically, 5 mg of the VIP derivative His-Ser-Asp-Ala-Val-Phe-Thr-Asp-Asn-Tyr-Thr-Arg-Leu-Arg-Arg-Gln-Met-Ala-Val-Arg-Arg-Tyr-Leu-Asn-Ser-Ile-Leu-Asn-Gly-Arg-Arg-NH₂ (SEQ ID NO: 1) as disclosed in Japanese Patent Publication (Unexamined Application) No. Hei-8-333276 was suitably mixed with 2 g of erythritol, and they were ground into fine particles using a jet mill under the conditions as specified in Example 1. Thus, a mixture of finely ground VIP derivative and erythritol was recovered from a bag filter for powder collection at a recovery rate of about 70%. It was confirmed by quantitative HPLC analysis that the VIP derivative content in the powders was almost equal to the theoretical value.

### [Example 7] Production of insulin powders

A mixture of insulin and erythritol (20-fold diluted powders) was finely ground using a jet mill as in Example 1 under the following conditions. Specifically, 100 mg of pig insulin (Itoham Foods Inc.) was suitably mixed with 1.9 g of erythritol, and they were ground into fine particles using a jet mill under the conditions as specified in Example 1. Thus, a mixture of finely ground insulin and erythritol was recovered from a bag filter for powder collection at a recovery rate of about 60%. It was confirmed by quantitative HPLC analysis that the insulin content in the powders was almost equal to the theoretical value.

### [Example 8] Production of salmon calcitonin powders

A mixture of salmon calcitonin and lactose (200-fold diluted powders) and a mixture of salmon calcitonin and erythritol (200-fold diluted powders) were finely ground using a jet mill as in Example 1 under the following conditions. Specifically, 15 mg of salmon calcitonin (Itoham Foods Inc.) was suitably mixed with about 3.0 g of lactose or erythritol, and they were ground into fine particles using a jet mill under the conditions as specified in Example 1. Thus, a finely ground powder mixture of salmon calcitonin was recovered from a bag filter for powder collection at recovery rates of about 70% (a mixture of salmon calcitonin and lactose) and about 80% (a mixture of salmon calcitonin and erythritol), respectively. It was confirmed by quantitative HPLC analysis that the salmon calcitonin content in the powders was almost equal to the theoretical value.

### [Example 9] Production of elcatonin powders

A mixture of elcatonin and lactose (200-fold diluted powders) and a mixture of elcatonin and erythritol (200-fold diluted powders) were finely ground using a jet mill as in Example 1 under the following conditions. Specifically, 15 mg of elcatonin (Itoham Foods Inc.) was suitably mixed with about 3.0 g of lactose or erythritol, and they were ground into fine particles using a jet mill under the conditions as specified in Example 1. Thus, finely ground powder mixture of elcatonin was recovered from a bag filter for powder collection at recovery rates of about 20% (a mixture of elcatonin and lactose) and about 70% (a mixture of elcatonin and erythritol). It was confirmed by quantitative HPLC analysis that the elcatonin content in the powders was almost equal to the theoretical value.

### [Example 10] Production of human growth hormone (GH) powders

A mixture of human growth hormone and erythritol (50-fold diluted powders) was finely ground using a jet mill as in Example 1 under the following conditions. Specifically, 30 mg of human growth hormone mixture (10 mg of human growth hormone, 10 mg of human albumin, and 10 mg of glycine, Jiangxi Chinese Import & Export Co., Ltd.) was suitably mixed with about 470 mg of erythritol, and they were ground into fine particles using a jet mill under the conditions as specified in Example 1. Thus, a mixture of finely ground human growth hormone and erythritol was recovered from a bag filter for powder collection at a recovery rate of about 50%. It was confirmed by quantitative HPLC analysis that the human growth hormone content in the powders was almost equal to the theoretical value. Also, the stability thereof was simultaneously confirmed. Thus, it was demonstrated that this method of preparing formulations could be applied to high molecular weight compounds such as human growth hormones, which were considered to be very unstable and difficult to handle.

### [Example 11] Production of cyclosporin powders

A mixture of cyclosporin and erythritol (10-fold diluted powders) was finely ground using a jet mill as in Example 1 under the following conditions. Specifically, 100 mg of cyclosporin A (Wako Pure Chemicals Industries, Ltd.) was suitably mixed with about 900 mg of erythritol, and they were ground into fine particles using a jet mill under the conditions as specified in Example 1. Thus, a mixture of finely ground cyclosporin and erythritol was recovered from a bag filter for powder collection at a recovery rate of about 60%. It was confirmed by quantitative HPLC analysis that the cyclosporin content in the powders was almost equal to the theoretical value.

### [Example 12] Production of citric acid-containing glucagon powders

A mixture of glucagon and citric acid and a mixture of glucagon, citric acid, and erythritol were finely ground respectively using a jet mill as in Example 1 under the following conditions. Specifically, 80 mg of glucagon (Itoham Foods Inc.) was mixed with 80 mg of citric acid. Alternatively, 80 mg of glucagon was suitably mixed with 80 mg of citric acid and about 640 mg of erythritol. They were ground into fine particles using a jet mill under the conditions as specified in Example 1. The finely ground mixture of glucagon and citric acid rapidly deliquesced and were very difficult to be recovered. In contrast, a mixture of glucagon, citric acid, and erythritol did not deliquesce due to moisture absorption and was very easy to handle. The recovery rate from a bag filter for powder collection was about 40%. This indicates that the formulation of the present invention was useful even with the inclusion of deliquescent additives. The glucagon content of the powders was confirmed to be almost equal to the theoretical value by quantitative HPLC analysis.

### [Example 13] Production of liposome-encapsulated glucagon

Glucagon (Itoham Foods Inc.) was prepared to 10 mg/mL with the aid of an aqueous solution of 0.1% trifluoroacetic acid, and the resulting solution was added in a dried empty liposome constituted by L-α-dipalmitoylphosphatidylcholine, cholesterol, and L-α-dipalmitoylphosphatidylglycerol (particle diameter: 100 to 300 nm, COATSOME EL series, NOF Corp.). The product was shaken for several minutes and then lyophilized (freeze-dryer Unitop HL, Freeze Mobile 25 EL, Virtis). About 200 µL of sample was collected before lyophilizing and centrifuged using a centrifugal ultrafiltration device for small amounts, Ultrafree-0.5 (removing substances having a molecular weight of 10,000 or lower), at 12,000 x g for 10 minutes. The glucagon content in the filtrate was quantified and this was determined to be the amount which was not encapsulated within a liposome. Conditions for quantitative HPLC analysis are as follows.

### (HPLC conditions)

| | |
|---|---|
| Column used: | TSK gel ODS-120T (TOSO) |
| Detector: | RF-535 (Shimadzu) |
| Excitation wavelength: | 280 nm |
| Fluorescence wavelength: | 346 nm |
| Pump: | LC-10 AD (Shimadzu) |
| Mobile phase: | 35% CH₃CN (0.1% TFA acidified) |
| Mobile phase flow rate: | 1.0 mL/min |

The result of quantification by HPLC demonstrated that the encapsulation rate of glucagon within liposome was very high at 98%.

### [Example 14] Production of liposome-encapsulated glucagon powders (1% glucagon powders)

About 800 mg of erythritol was suitably mixed with about 200 mg of liposome-encapsulated glucagon obtained by the method as described in Example 13, and they were finely ground using a jet mill as in Example 1. A finely ground mixture of liposome-encapsulated glucagon and erythritol was recovered from a bag filter for powder collection at a recovery rate of about 50%. The resultant powders have high fluidity and are good particles from the viewpoint of formulation properties. It was confirmed by quantitative HPLC analysis that the glucagon content in the powders was almost equal to the theoretical value. This indicates that this method of preparing formulations can be applied to liposome formulations, and liposome formulations can be produced without the deliquescence of medicaments during manufacturing.

### [Example 15] Mixing of powders and carriers (a Reference Example)

Each of the lactose powders and the 1 % glucagon-lactose powders obtained in Example 2 was rapidly mixed with carriers (Pharmatose 325 M, DMV, average particle diameter 50 ± 10 µm) under the conditions as specified in Table 4 and dried and stored in a 50 mL disposable tube, respectively. Properties of the powders that were visually observed in that case were also shown in Table 4. When the lactose powders and the 1 % glucagon-lactose powders were added to the carriers, they had many properties in common, including their fluidities. This indicated that an undesirable property of glucagon, i.e., self-aggregation, could be inhibited. On the other hand, finely ground 1 % glucagon-lactose powders, which were not mixed with carriers, form clear aggregates at least 24 hours later.

As is apparent from these results, the powder formulations according to the present invention were very suitable for administration because of inhibited self-aggregation.

**Table 4**

| (Lactose) | | |
|---|---|---|
| | Carrier: Ground lactose | Properties |
| (i) | 1.0 : 0.1 | Substantially same fluidity as carrier |
| (ii) | 1.0 : 0.2 | Substantially same fluidity as carrier |
| (iii) | 1.0 : 0.5 | Substantially same fluidity as carrier, |
| (iv) | 1.0 : 0.75 | Adhered on the wall due to excessive amount of lactose |
| (v) | 1.0: 1.0 | Poor fluidity due to excessive amount of lactose |

| (1 % glucagon-lactose powder) | | |
|---|---|---|
| | Carriers: Ground 1% powders | Properties |
| (i) | 1.0 : 0.1 | Substantially same fluidity as carrier |
| (ii) | 1.0; 0.2 | Substantially same fluidity as carrier |
| (iii) | 1.0:0.5 | Substantially same fluidity as carrier |

### [Example 16] Particle size distribution of mixture of carriers and 1 % glucagon ground powders (a Reference Example)

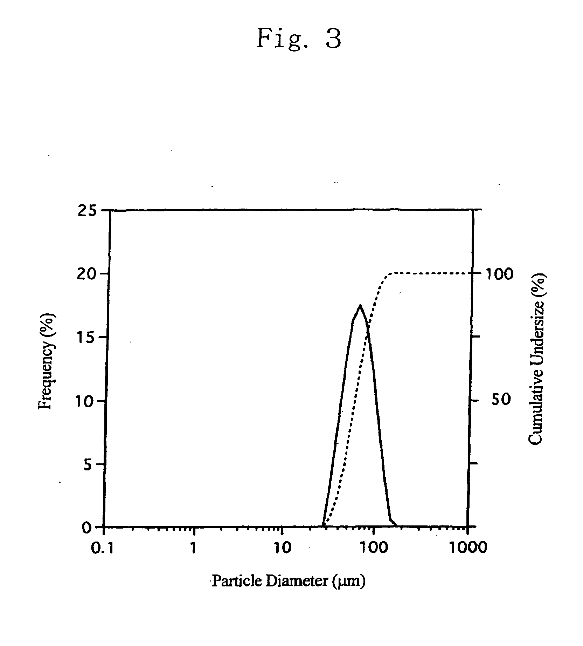

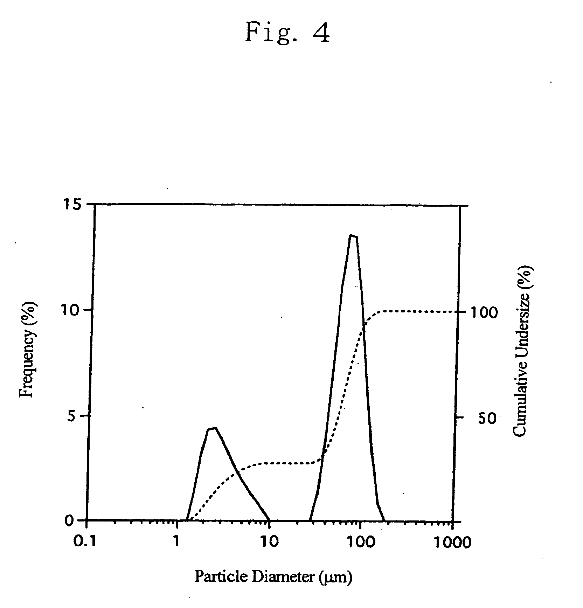

A particle size distribution of carriers (Pharmatose 325 M) only and that of a mixture of carriers and 1 % glucagon ground powders were examined using the SK LASER MICRON SIZER LMS-300 (Seishin Enterprise Co., Ltd.) (Figs. 3 and 4). As a result, when powders were allowed to disperse at the pressure of 2.0 kg/cm², carriers were apparently separated from medicaments as shown in Fig. 4. In addition, the separated 1 % glucagon powders did not exhibit any increase in particle sizes due to self-aggregation.

As is apparent from this result, the powder formulations according to the present invention do not undergo self-aggregation. At the time of administration, medicaments are easily separated from carriers and only the fine medicament-containing particles are able to reach the target site.

### [Example 17] Evaluation of uniformility of glucagon content (a Reference Example)

After mixing with carriers, the uniformity of medicament content is presumed to improve markedly by inhibiting self-aggregation of glucagon and improving its fluidity. Accordingly, medicaments were randomly sampled from the powders obtained in Example 16 and subjected to quantitative HPLC analysis to evaluate the uniformity. The process is shown below.

Conditions for high-performance liquid chromatography (HPLC) analysis on glucagon containing formulations are as described below.

### (Conditions for HPLC analysis)

| | |
|---|---|
| Column used: | TOSO ODS-120T(TOSO) |
| Detector: | Jusco 870-UV |
| UV detection wavelength: | 220 nm |
| Mobile phase flow rate: | 1.0mL/min |
| Mobile phase: | 35% CH₃CN aq (0.1% TFA acidified) |
| Pump: | Jusco 880-PU |
| Amount injected: | 50 µL |
| Column oven temperature: | 40°C |

### (Method)

### Object sample

A mixture of glucagon powders (DPG080700) and Pharmatose
(mixing ratio: 100% glucagon-lactose powders : carriers = 1 : 2)

This specimen (volume: 25 mL) was divided into three substantially equal sections in a 50 mL disposable tube, and about 10 mg each of 3 specimens (samples 1 to 3) were randomly sampled from each section. These specimens were prepared to 10 mg/mL with the aid of 0.1N HCl and analyzed by RP-HPLC. The peak areas corresponding to glucagon were then compared among specimens.

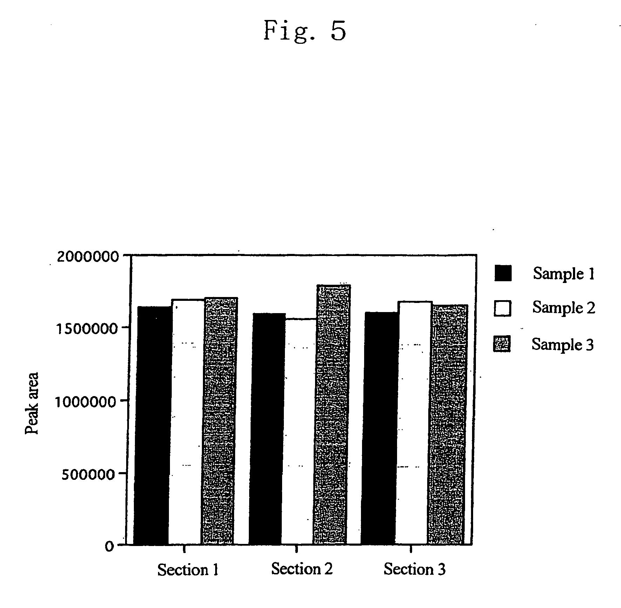

Fig. 5 shows peaks corresponding to glucagon in each section for samples.

As is apparent from Fig. 5, substantially equivalent amounts of medicaments were detected in randomly collected samples, and the powder formulations according to the present invention have improved fluidity and are almost uniformly mixed with carriers.

### [Example 18] Evaluation of glucagon and elcatonin formulations using cascade impactor (a Reference Example)

In order to investigate aerodynamic particle diameters of powders, examination was carried out using a cascade impactor as a model of artificial airways and lung. The main body comprises 8 stages and a final filter superposed on top of another in combination with a flow meter and a suction pump. The method described in "Multistage Cascade Impactor Apparatus" in USP 2000 "Physical Tests and Determinations/Aerosols" was basically employed. A specific condition is as described below.

### (Method)

| | |
|---|---|
| Apparatus: | Andersen sampler (AN-200, Sibata Scientific Technology Ltd.) |
| Pump flow rate: | 28.3 L/min |
| Device used: | Jet-haler (UNISIA JECS) |
| Specimens: | Mixture of glucagon powders and Pharmatose |
| | (mixing ratio: 1% glucagon-lactose powders : carriers = 1 : 2) |
| | Mixture of elcatonin powders and Pharmatose |
| | (mixing ratio: 1 % elcatonin-lactose powders: carriers = 1 : 2) |

Suitable amounts of the formulations were filled into the No. 2 capsules specified by the Japanese Pharmacopoeia and placed on the device.

### Quantification of medicaments: Conditions for HPLC described in Example 17

### (Results)

Quantified values by HPLC are shown below.

**Table 5**

| Glucagon formulations | | |
|---|---|---|
| Sampling stage | Amount of glucagon collected (µg) | Accumulated glucagon (%) |
| Stage 0 | 72.28 | 100.0 |
| Stage 1 | 0.60 | 32.6 |
| Stage 2 | 0.50 | 32.0 |
| Stage 3 | 4.30 | 31.6 |
| Stage 4 | 21.89 | 27.6 |
| Stage 5 | 7.06 | 7.1 |
| Stage 6 | 0.47 | 0.6 |
| Stage 7 | 0.13 | 0.1 |

**Table 6**

| Elcatonin formulations | | |
|---|---|---|
| Sampling stage | Amount of elcatonin collected (µg) | Accumulated elcatonin (%) |
| Stage 0 | 3.08 | 100.0 |
| Stage 1 | 0.14 | 42.3 |
| Stage 2 | 0.80 | 39.6 |
| Stage 3 | 0.78 | 24.5 |
| Stage 4 | 0.36 | 9.8 |
| Stage 5 | 0.08 | 3.0 |
| Stage 6 | 0.07 | 1.4 |
| Stage 7 | 0 | 0 |

According to this model, medicaments collected at stage 3 or later would migrate to the bronchi and lungs. Since about 30% to 40% of main components are collected at stage 3 or later for both formulations, it is expected that about 30% to 40% of medicaments that enter into the body would take effect preferably.

### [Example 19] Influence of the mixing ratio of carriers to fine medicament particles on formulation properties (a Reference Example)

The lactose listed in the Japanese Pharmacopoeia (Yoshida Pharmaceutical Co., Ltd.) was finely ground in the same manner as described in Example 1, and the resulting powders were mixed with carriers (Pharmatose 325 M, DMV) using an unelectrified bag under the following conditions to prepare formulations for DPIs.

**Table 7**

| | Carrier : Lactose powder |
|---|---|
| (i) | 1.0:0.1 |
| (ii) | 1.0:0.2 |
| (iii) | 1.0:0.5 |

These formulations were analyzed using a cascade impactor. Fundamental conditions for analysis are as in Example 18. RF values are shown in Table 8. These RF values are obtained by weighing the powders collected at each stage and dividing the amounts collected at stages 2 to 5 by a total amount of finely ground substances contained in the formulations.

**Table 8**

| | RF value |
|---|---|
| (i) | 44.9% |
| (ii) | 30.5% |
| (iii) | 18.1% |

These results indicate that the mixing ratio of excipients to particles is significantly involved with the formulation properties, and that low medicament content towards carriers is preferable.

### [Example 20] Evaluation of content uniformity of liposome-encapsulated glucagon formulations

Powders of liposome-encapsulated glucagon produced in Example 14 were mixed with carriers (Pharmatose 325 M, DMV) using an unelectrified bag to produce formulations for DPIs. In accordance with the method described in Example 17, specific amounts of samples were weighed and sampled from three sections, and the glucagon contents therein were quantified by HPLC. Conditions for HPLC are as shown below.

### (Conditions for HPLC)

| | |
|---|---|
| Column used: | TOSO ODS-120T (TOSO) |
| Detector: | RF-535 (Shimadzu) |
| Excitation wavelength: | 280 nm |
| Fluorescence wavelength: | 346 nm |
| Mobile phase flow rate: | 1.0 mL/min |
| Mobile phase: | 35% CH₃CN aq (0.1% TFA acidified) |
| Pump: | LC-10 AD (Shimadzu) |
| Column oven temperature: | 40°C |

The specimens sampled from the three sections were quantified under the above conditions, and as a result, glucagon contents were respectively 2.32 µg/mg of formulation, 2.43 µg/mg of formulation, and 2.41 µg/mg of formulation, with an average content of 2.39 µg/mg of formulation. Even when molecules such as liposomes, which were likely to aggregate and associate, were used as in this case, the method of preparing formulations according to the present invention was found to be capable of providing formulations having approximately uniform medicament contents.

### [Example 21] Evaluation of content uniformity of salmon calcitonin formulations

Powders of salmon calcitonin produced in Example 8 were mixed with carriers (Pharmatose 325 M, DMV) using an unelectrified bag to produce formulations for DPIs. In accordance with the method described in Example 17, specific amounts of samples were weighed and sampled from the three sections, and the salmon calcitonin contents therein were quantified by HPLC. Conditions for HPLC are as shown below.

### (Conditions for HPLC)

| | |
|---|---|
| Column used: | TOSO ODS-120T (TOSO) |
| Detector: | RF-535 (Shimadzu) |
| Excitation wavelength: | 280 nm |
| Fluorescence wavelength: | 320 nm |
| Mobile phase flow rate: | 1.0 mL/min |
| Mobile phase: | 38% CH₃CN aq (0.1 % TFA acidified) |
| Pump: | Jusco 880-PU |
| Column oven temperature: | 40°C |

The specimens sampled from the three sections were quantified under the above conditions, and as a result, salmon calcitonin contents were respectively 3.95 µg/mg of formulation, 4.29 µg/mg of formulation, and 4.05 µg/mg of formulation, with an average content of 4.09 µg/mg of formulation. Even when the medicament contents were extremely low as with this case, the method of preparing formulations according to the present invention was found to be capable of providing formulations having approximately uniform medicament contents.

### [Example 22] Evaluation of glucagon formulations using cascade impactor

Glucagon powders produced by the method described in Example 3 were mixed with carriers (Pharmatose 325 M, DMV) using an unelectrified bag to produce formulations for DPIs. These formulations were analyzed using a cascade impactor. Fundamental conditions for analysis are as in Example 18. RF values and retention in capsules are shown in Table 9. These RF values are obtained by quantifying by HPLC the glucagon in formulations collected at each stage and dividing the amounts of glucagon at stages 2 to 5 by the total amount of glucagon contained in the encapsulated formulations. Conditions for HPLC analysis are as shown below.

### (Object formulations)

A mixture of 1 % glucagon and lactose powders, and lactose carrier

A mixture of 1 % glucagon and erythritol powders, and lactose carrier

### (Conditions for HPLC analysis)

| | |
|---|---|
| Column used: | ODS-120T (TOSO) |
| Detector: | RF-535 (Shimadzu) |
| Pump: | LC-10 AD (Shimadzu) |
| Excitation wavelength: | 280 nm |
| Fluorescence detection wavelength: | 346 nm |
| Mobile phase flow rate: | 1.0 mL/min |
| Mobile phase: | 35% CH₃CN aq (0.1 % TFA acidified) |
| Column oven temperature: | Room temperature |

This analysis revealed the characteristics of release from the capsules, subsequent dispersibility, and ease of dissociation from carriers of formulations using lactose and formulations using erythritol.

**Table 9**

| | RF value | Retention in capsule |
|---|---|---|
| Glucagon | 0% | Almost all formulations remained due to the aggregate formation |
| Mixed formulation of glucagon-lactose | 6.4% | Two out of four remained after suctioning for 10 seconds twice |
| Mixed formulation of glucagon-erythritol | 56.7% | All formulations were released after suctioning for 10 seconds |

### [Example 23] Evaluation of formulations containing glucagon at high level using cascade impactor

The powder mixture of glucagon and erythritol produced by the method described in Example 3 were mixed with carriers (Pharmatose 325 M, DMV) using an unelectrified bag to produce formulations for DPIs. These formulations were analyzed using a cascade impactor. Fundamental conditions for analysis are as in Example 18. RF values are shown in Table 10. These RF values are obtained by quantifying by HPLC the glucagon in formulations collected at each stage and dividing the amounts of glucagon at stages 2 to 5 by a total amount of glucagon contained in the encapsulated formulations. Conditions for HPLC analysis are as in Example 22.

**Table 10**

| Dilution of glucagon powder | RF value |
|---|---|
| 1-fold | 18.6% |
| 5-fold | 69.4% |
| 10-fold | 42.9% |
| 20-fold | 41.2% |

This result demonstrates the effect of the addition of excipients. It was confirmed that formulation of 5-fold diluted peptide powders had good formulation properties even under such high concentration conditions. As recognized in Example 22, in the absence of excipients, it is very difficult to provide formulations that can be used clinically. Thus, the usefulness of the formulations according to the present invention is strongly indicated.

### [Example 24] Evaluation of citric acid-containing glucagon formulations using cascade impactor

The powder mixture of glucagon, citric acid, and erythritol produced by the method described in Example 12 (10% glucagon powders) was mixed with carriers (Pharmatose 325 M, DMV) using an unelectrified bag to produce formulations for DPIs. These formulations were analyzed using a cascade impactor. RF values are shown in Table 11. These RF values are obtained by quantifying by HPLC the glucagon in formulations collected at each stage and dividing the amounts of glucagon at stages 2 to 5 by the total amount of glucagon contained in the encapsulated formulations. Conditions for HPLC analysis are as in Example 17.

**Table 11**

| Formulation | RF value |
|---|---|
| Glucagon-citric acid | Unconfirmed due to deliquescence |
| Glucagon-erythritol-citric acid | 28.3% |

In addition to peptides and proteins, many compounds as additives are pharmaceutically difficult to handle. Citric acid that was used in this case was one of such example, and it rapidly deliquesced in the absence of erythritol. Thus, it has a very serious problem in terms of clinical application. According to the method of preparing formulations of the present invention, however, very beneficial formulation properties can be demonstrated even in the presence of citric acid, and peptide medicaments with extremely high concentration as in this example.

### [Example 25] Evaluation of liposome-encapsulated glucagon formulations using cascade impactor

The liposome-encapsulated glucagon powders produced by the method described in Example 14 were mixed with carriers (Pharmatose 325 M, DMV) using an unelectrified bag to produce formulations for DPIs. These formulations were analyzed using a cascade impactor. Fundamental conditions for analysis are as in Example 18. The weights of powders collected at each stage were measured and as a result about 30% of fine particles were collected at stages 2 to 5, which represented conditions equivalent to those of bronchi or lungs. Liposome-encapsulated medicaments, which consist of lipids, are likely to form aggregates due to the properties of lipids. As demonstrated in this example, however, a rate of medicaments reaching the lungs that can be clinically used can be attained.

### [Example 26] Evaluation of VIP derivative formulations using cascade impactor

The powder mixture of VIP derivative and erythritol (400-fold diluted powder of VIP derivatives) produced by the method described in Example 6 was mixed with carriers (Pharmatose 325 M, DMV) using an unelectrified bag to produce formulations for DPIs. These formulations were analyzed using a cascade impactor. Fundamental conditions for analysis are as in Example 18. The weights of powders collected at each stage were measured and as a result about 40% of fine particles were collected at stages 2 to 5, which represented conditions equivalent to those of the bronchi or lungs.

### [Example 27] Evaluation of insulin formulations using cascade impactor

The powder mixture of insulin and erythritol produced by the method described in Example 7 was mixed with carriers (Pharmatose 325 M, DMV) using an unelectrified bag to produce formulations for DPIs. These formulations were analyzed using a cascade impactor. Fundamental conditions for analysis are as in Example 18. Conditions for HPLC analysis are as shown below.

### (Conditions for HPLC analysis)

| | |
|---|---|
| Column used: | ODS-120T (TOSO) |
| Detector: | SPD-10 A (Shimadzu) |
| Pump: | LC-10 AD (Shimadzu) |
| Detection wavelength: | 220 nm |
| Mobile phase flow rate: | 1.0 mL/min |
| Mobile phase: | 34% CH₃CN aq (0.1 % TFA acidified) |
| Column oven temperature: | Room temperature |

As a result of this analysis, the RF value of insulin formulation was about 20%, and this insulin formulation for inhalation was sufficient for clinical applications.

### [Example 28] Evaluation of salmon calcitonin formulations using cascade impactor

The powder mixture of salmon calcitonin and lactose and the powder mixture of salmon calcitonin and erythritol produced by the method described in Example 8 were mixed with carriers (Pharmatose 325 M, DMV) using an unelectrified bag to produce formulations for DPIs. These formulations were analyzed using a cascade impactor. Fundamental conditions for analysis are as in Example 18. RF values calculated after the analysis are shown in Table 12. These RF values are obtained by quantifying by HPLC the calcitonin in formulations collected at each stage and dividing the amounts of calcitonin at stages 2 to 5 by a total amount of calcitonin contained in the encapsulated formulations. Conditions for HPLC analysis are as shown below.

**Table 12**

| | RF value |
|---|---|
| | (when the amount of calcitonin contained in the formulation is determined to be 100%) |
| Mixed formulation of salmon calcitonin and lactose | 29.9% |
| Mixed formulation of salmon calcitonin and erythritol | 52.0% |

This analysis revealed the characteristics of release from the capsules, subsequent dispersibility, and ease of dissociation from carriers of formulations using lactose and formulations using erythritol.

### [Example 29] Evaluation of elcatonin formulations using cascade impactor

The powder mixture of elcatonin and erythritol produced by the method described in Example 9 was mixed with carriers (Pharmatose 325 M, DMV) using an unelectrified bag to produce formulations for DPIs. These formulations were analyzed using a cascade impactor. Fundamental conditions for analysis are as in Example 18. The weights of powders collected at each stage were measured and as a result about 40% of fine particles were collected at stages 2 to 5, which represented conditions equivalent to those of bronchi or lungs.

### [Example 30] Evaluation of growth hormone (GH) formulations using cascade impactor

The powder mixture of growth hormone (GH) and erythritol produced by the method described in Example 10 was mixed with carriers (Pharmatose 325 M, DMV) using an unelectrified bag to produce formulations for DPIs. These formulations were analyzed using a cascade impactor. Fundamental conditions for analysis are as in Example 18. The weights of powders collected at each stage were measured and as a result about 47% of fine particles were collected at stages 2 to 5, which represented conditions equivalent to those of bronchi or lungs.

### [Example 31] Evaluation of cyclosporin formulations using cascade impactor

The powder mixture of cyclosporin and erythritol produced by the method described in Example 11 was mixed with carriers (Pharmatose 325 M, DMV) using an unelectrified bag to produce formulations for DPIs. These formulations were analyzed using a cascade impactor. Fundamental conditions for analysis are as in Example 18. The weights of powders collected at each stage were measured and as a result about 40% of fine particles were collected at stages 2 to 5, which represented conditions equivalent to those of bronchi or lungs.

### [Example 32] Evaluation of ground excipients using cascade impactor

Various excipient powders produced by the method described in Example 1 (lactose, mannitol, and erythritol) were mixed with carriers (Pharmatose 325 M, DMV) using an unelectrified bag to produce formulations for DPIs.

These formulations for DPIs were analyzed using a cascade impactor as a model of artificial airways and lungs in accordance with the method as described in the United States Pharmacopeia to examine the dispersibility and the rate of reaching the target tissues for various samples. The method for analysis is as described in Example 18.

| | |
|---|---|
| Specimens: | Mixture of ground lactose and lactose carrier |
| | Mixture of ground erythritol and lactose carrier |
| | Mixture of ground mannitol and lactose carrier |

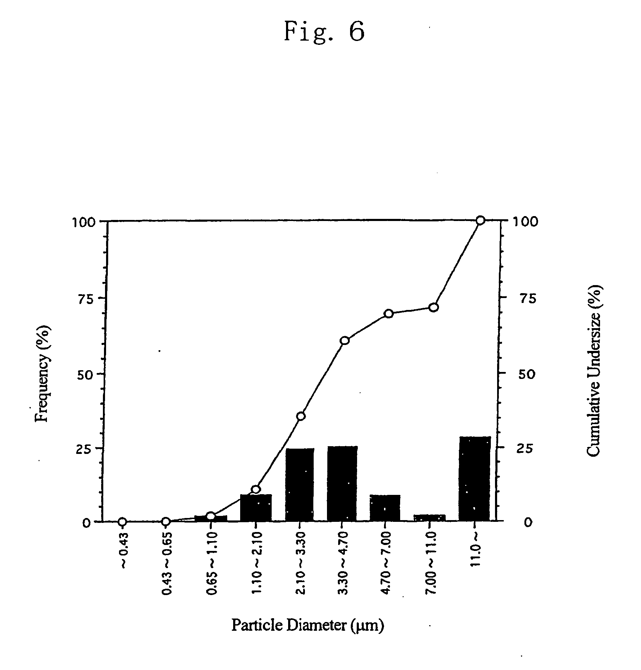

The analysis results on the mixture of finely ground excipients and carriers using a cascade impactor are shown in Figs. 6 to 8 (the bar chart indicates the percentage of the collected amount at each stage relative to the total collected amount in the device, and the line chart indicates the percentage of accumulated amounts).

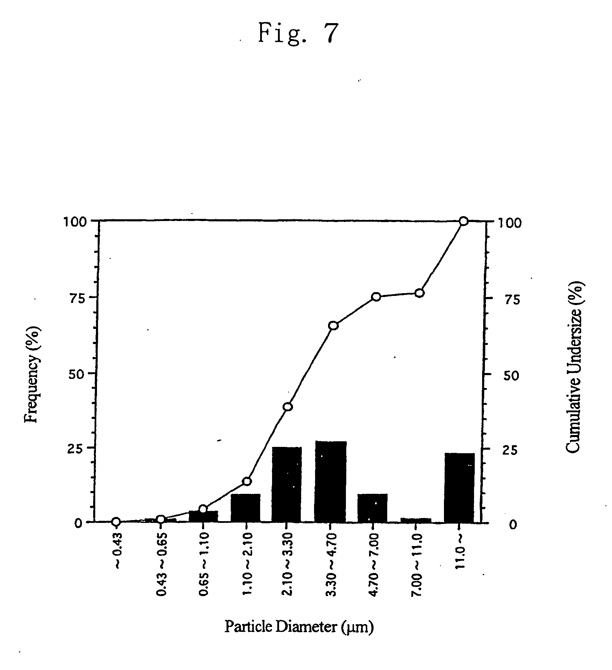

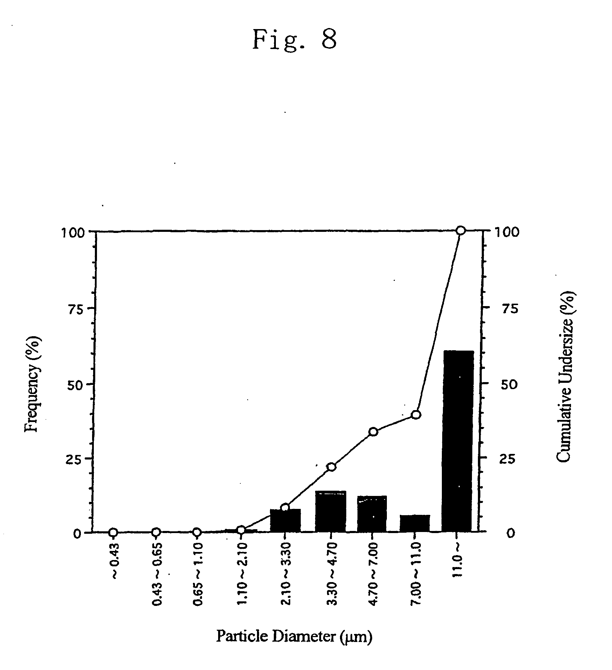

Fig. 6 shows the results on the erythritol mixture, Fig. 7 shows the results on the mannitol mixture, and Fig. 8 shows the results on the lactose mixture. As is apparent from these results, ground erythritol and ground mannitol were low in cohesion with a lactose carrier and thus easily separated from the carrier. What is notable is a low-hygroscopic property of erythritol, and this can significantly inhibit weight changes with the elapse of time. This is considered to significantly contribute to the improvement in formulation properties.

### [Example 33] Properties of buserelin acetate formulations using different excipients

The formulations containing buserelin acetate for inhalation produced in Example 4 were analyzed using a cascade impactor to compare properties of each formulation. The fundamental method for analysis is as in Example 18.

| | |
|---|---|
| Device used: | Jet-haler (UNISIA JECS) |
| Specimens: | Mixed formulation of ground powders of 570-fold diluted buserelin with lactose and lactose carrier |
| Mixed formulation of ground powders of 570-fold diluted buserelin with erythritol and lactose carrier | |
| Mixed formulation of ground powders of 570-fold diluted buserelin with mannitol and lactose carrier Suitable amounts of the formulations were filled into the No. 2 capsule specified by the Japanese Pharmacopoeia and placed on the device. | |
| Suction time: | Basically 10 seconds. When retention in capsules is observed, suction was conducted again for 10 seconds. |

As a result of analysis using a cascade impactor, formulations using erythritol as an excipient had very high characteristics of release from capsules and exhibited high RF values, as shown in Table 13. Also, mannitol was poor in fluidity and the amount of formulation remaining in the capsule was relatively high. Thus, it is presumed to be inevitable that the RF values are low.

**Table 13**

| | RF value | Retention in capsule |
|---|---|---|
| Mixed formulation of buserelin-lactose | 11.3% | Remained after suctioning for 10 seconds twice |
| Mixed formulation of buserelin-erythritol | 29.3% | All formulations were released after suctioning for 10 seconds |
| Mixed formulation of buserelin-mannitol | 8.02% | Remained after suctioning for 10 seconds twice |

### [Example 34] Properties of glucagon formulations using different excipients

The glucagon formulations produced by the method described in Example 3 were analyzed using a cascade impactor. Fundamental conditions for analysis are as in Example 18. RF values and retention in capsules are shown in Table 14. These RF values are obtained by quantifying by HPLC the glucagon in formulations collected at each stage and dividing the amounts of glucagon at stages 2 to 5 by a total amount of glucagon contained in the encapsulated formulations. Conditions for HPLC analysis are as shown below.

### (Object formulations)

A mixture of 1 % glucagon powder with lactose, and a lactose carrier [(finely ground substance) / (325 M) = 0.4]

A mixture of 1 % glucagon powder with erythritol, and a lactose carrier [(finely ground substance) / (325 M) = 0.2]

### (Conditions for HPLC analysis)

| | |
|---|---|
| Column used: | TOSO ODS-120T (TOSO) |
| Detector: | RF-535 (Shimadzu) |
| Pump: | LC-10 AD (Shimadzu) |
| Excitation wavelength: | 280 nm |
| Fluorescence detection wavelength: | 346 nm |
| Mobile phase flow rate: | 1.0 mL/min |
| Mobile phase: | 35% CH₃CN aq (0.1% TFA acidified) |
| Column oven temperature: | Room temperature |

This result revealed the characteristics of release from the capsules, subsequent dispersibility, and ease of dissociation from carriers of formulations using lactose and formulations using erythritol.

**Table 14**

| | RF values | |
|---|---|---|
| | (when the amount of glucagon contained in the formulation is determined to be 100%) | Retention in capsule |
| Mixed formulation of glucagon-lactose | 6.4% | Two out of four remained after suctioning twice for 10 seconds each |
| Mixed formulation of glucagon-erythritol | 56.7% | All formulations were released after suctioning for 10 seconds |

### [Example 35] Rate of absorbing glucagon formulations through a rat lung

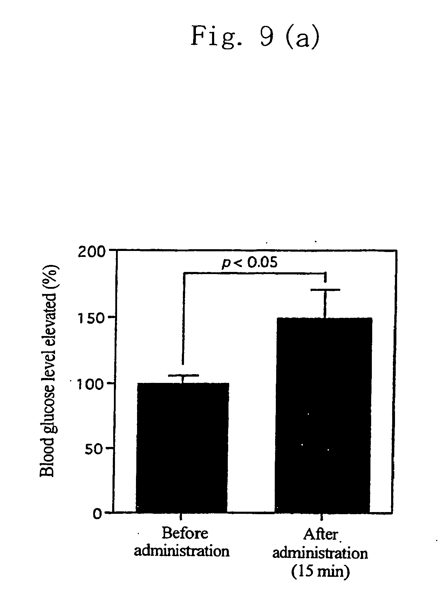

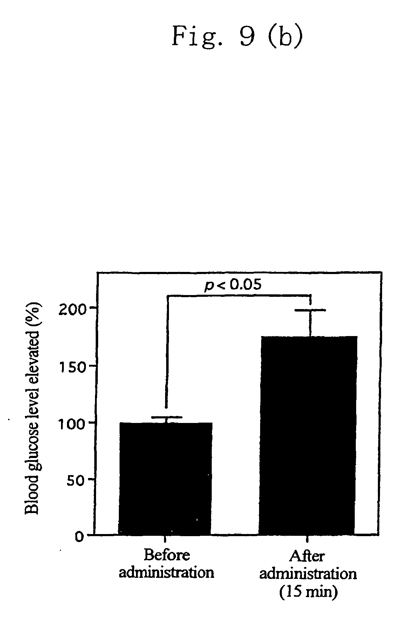

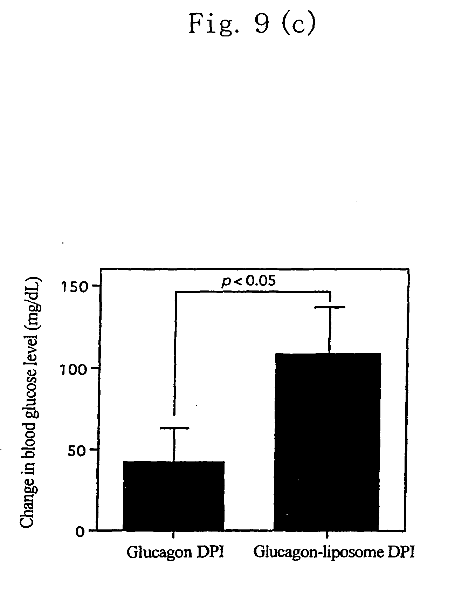

The glucagon formulations for DPIs produced in Example 3 and the liposome-encapsulated glucagon formulations for DPIs produced in Example 14 were administered to experimental animals in the following manner, and their blood glucose levels were monitored with the elapse of time. Various glucagon formulations were administered to the lungs of male SD rats with body weights of 300 to 400 g under Nembutal anesthesia using a device for pulmonary administration. After the administration, about 1 mL of blood was sampled through the jugular vein with the elapse of time, 4.8 mg of EDTA (2 Na) was added, and centrifugation was conducted to obtain a plasma sample. The plasma was immediately stored at -40°C. The glucose level in the plasma was calculated by the enzyme method. Fig. 9 (a) shows changes in blood glucose levels by tranpulmonary administration of glucagon formulations by DPIs that are not encapsulated within liposome. Fig. 9(b) shows the result of administration of liposome-encapsulated glucagon by DPIs. In both cases, blood glucose levels are significantly elevated from those before the administration. What is further notable is a more potent effect of liposome-encapsulated glucagon administered by DPIs as shown in Fig. 9 (c).

### [Example 36] Production of powders of various compounds

All the compounds were finely ground in the same manner using aerodynamic mill.

### Grinding conditions

| | |
|---|---|
| Equipment used: | A-O-Jet Mill (Seishin Enterprise Co., Ltd.) |
| Means for feeding crude materials: | Automatic feeder |
| Feeding air pressure: | 6.0 kg/cm² G |
| Grinding air pressure: | 6.5 kg/cm² G |

### Dust collection: Collecting bag (polyethylene)

In the above method, ground glucagon, salmon calcitonin, insulin lactose, erythritol, theophylline, or tranilast was produced, and they were mixed with an erythritol carrier (average particle size: about 70 µM) or a lactose carrier (average particle size: about 50 µm) using an unelectrified bag [(finely ground substance) / (325 M) = 0.4]. Yields were about 50% to 70%. [Example 37] Release characteristics of glucagon formulations

The glucagon formulations produced in Example 36 were analyzed using a cascade impactor for the dispersibility of various samples and their rates of reaching the target tissues.

### Analysis method using cascade impactor

| | |
|---|---|
| Apparatus: | Andersen sampler (AN-200, Sibata Scientific Technology Ltd.) |
| Pump flow rate: | 28.3 L/min |
| Specimens: | Ground glucagon-lactose + lactose carrier |
| | Ground glucagon-lactose + erythritol carrier |
| | Ground glucagon-erythritol + lactose carrier |
| | Ground glucagon-erythritol + erythritol carrier |

These formulations were filled into the No. 2 capsules specified by the Pharmacopoeia of Japan and analyzed using the following device.

| | |
|---|---|
| Device used: | Jet-haler (UNISIA JECS) |

### Conditions for HPLC analysis

The amount of glucagon collected at each stage by the analysis using a cascade impactor was subjected to quantification by HPLC under the following conditions.

| | |
|---|---|
| Column used: | TOSO ODS-120T (TOSO) |
| Detector: | RF-535 (Shimadzu) |
| Pump: | LC-10 AD (Shimadzu) |
| Excitation wavelength: | 280 nm |
| Fluorescence detection wavelength: | 346 nm |
| Mobile phase flow rate: | 1.0 mL/min |
| Mobile phase: | 35% CH₃CN aq (0.1% TFA acidified) |
| Column oven temperature: | Room temperature |

Fig. 10 shows the result of analysis using a cascade impactor (the line chart indicates the percentage of the amount of collected glucagon at each stage relative to the total glucagon amount in the formulations). According to Fig. 10, the amount of glucagon collected increases more with the use of erythritol as an excipient or carrier than with the use of lactose as a carrier and an excipient. Further, when erythritol is used as a carrier and an excipient, the amount of glucagon collected is the highest. This result clearly demonstrates the usefulness of an erythritol carrier. Also, it was demonstrated that the formulation properties were further improved by using erythritol also as an excipient. It is notable that the collected amount at stage 0 (particle size: not less than 11 µM) which is equivalent to the throat in this model obviously decreased with the use of erythritol. This can inhibit, for example, local accumulation in the throat or local irritation that could be caused by the medicaments.

Table 15 shows the rate of glucagon formulations migrating to the lung in this model.

**Table 15**

| | | Kinds of carrier | |
|---|---|---|---|
| | | Lactose | Erythritol |
| Kinds of excipients | Lactose | 6.4% | 33.9% |
| | Erythritol | 37.1% | 59.8% |

As is apparent from the results shown in Table 15, there were clear differences in the rates of formulations reaching the lungs between formulations using a lactose carrier and formulations using an erythritol carrier. This was found not only with the use of erythritol as a carrier but also with the use of erythritol as an excipient. When both carriers and excipients were erythritol, much better results, which were far beyond the above values, were obtained. Based on this result, the usefulness of the use of erythritol as a carrier and the synergistic effect obtained with the use of erythritol as an excipient were confirmed.

### [Example 38] Release characteristics of insulin formulation

The insulin formulations produced in Example 1 were analyzed using a cascade impactor for the dispersibility of various samples and their rates of reaching the target tissues.

| | |
|---|---|
| Specimens: | Ground insulin-lactose + lactose carrier |
| | Ground insulin-lactose + erythritol carrier |

Both formulations were filled into the No. 2 capsules specified by the Pharmacopoeia of Japan and analyzed using the Jet-haler.

### Conditions for HPLC analysis

The amount of glucagon collected at each stage by the analysis using a cascade impactor was subjected to quantification by HPLC under the following conditions.

| | |
|---|---|
| Column used: | TOSO ODS-120T (TOSO) |
| Detector: | SPD-10 A (Shimadzu) |
| Pump: | LC-10 AD (Shimadzu) |
| Detection wavelength: | 220 nm |
| Mobile phase flow rate: | 1.0 mL/min |
| Mobile phase: | 34% CH₃CN aq (0.1% TFA acidified) |
| Column oven temperature: | Room temperature |

As a result of analysis under the above conditions, the rates of medicaments reaching the lungs and the bronchi were calculated as shown in Table 16. As is apparent from the results shown in Table 16, when erythritol was used as a carrier, the rates of medicaments reaching the lungs and the bronchi were much higher than those attained when lactose was used as a carrier. This indicates that erythritol is useful as a carrier.

**Table 16**

| | Kinds of carrier | |
|---|---|---|
| | Lactose | Erythritol |
| Rate of medicaments reaching lungs and bronchi | 14.6% | 22.2% |

### [Example 39] Release characteristics of salmon calcitonin formulations

The salmon calcitonin formulations produced in Example 36 were analyzed using a cascade impactor for the dispersibility of various samples and their rates of reaching the target tissues.

| | |
|---|---|
| Specimens: | Ground salmon calcitonin-lactose + lactose carrier |
| | Ground salmon calcitonin-lactose + erythritol carrier |

Both formulations were filled into the No. 2 capsules specified by the Pharmacopoeia of Japan and analyzed using the Jet-haler.

The amounts of salmon calcitonin formulations collected at each stage were measured and the rates of medicaments reaching the lungs and the bronchi were calculated. As a result, the results shown in Table 17 were obtained. As with the results obtained in Example 38, a high rate of medicaments reaching the lungs and the bronchi was achieved when erythritol was used as a carrier, and the usefulness of an erythritol carrier was also found for salmon calcitonin.

**Table 17**

| | Kinds of carrier | |
|---|---|---|
| | Lactose | Erythritol |
| Rate of medicaments reaching lungs and bronchi | 29.9% | 61.8% |

### [Example 40] Release characteristics of tranilast formulations

The tranilast formulations produced without using an excipient in Example 36 were analyzed using a cascade impactor in the same manner as in Examples 37 to 39 for the dispersibility of various samples and their rates of reaching the target tissues. Specimens: Ground tranilast + lactose carrier Ground tranilast + erythritol carrier

Both formulations were filled into the No. 2 capsules specified by the Pharmacopoeia of Japan and analyzed using the Jet-haler.
The amounts of tranilast formulations collected at each stage were measured and, as a result, the rates of medicaments reaching the lungs and the bronchi were calculated as shown in Table 18.

**Table 18**

| | Kinds of carrier | |
|---|---|---|
| | Lactose | Erythritol |
| Rate of medicaments reaching lungs and bronchi | 11.4% | 45.5% |

As is apparent from the results shown in Table 18, while the rate of reaching the target tissue is somewhat low with the use of a lactose carrier, the rate of reaching the target tissue is remarkably enhanced with the use of an erythritol carrier. The tranilast used in this analysis is not a peptide or protein pharmaceutical. Specifically, it was demonstrated that an erythritol carrier was very effective for pharmaceuticals other than peptide and protein pharmaceuticals.

### Industrial Applicability

The powder formulations according to the present invention can be provided as formulations with content uniformity by inhibiting peptides and proteins, which are very useful in living organisms, from causing self-aggregation under the specific conditions. In addition, the formulations can be clinically provided without deterioration of the properties of contained pharmaceutical compounds. They are also effective from the viewpoint of medical economics.

Further, the use of sugar alcohols containing erythritol i.e., a low hygroscopic and low cohesive compound, as carriers facilitates the dissociation of fine medicament-containing particles from carriers and improves properties of powder formulations such as tissue selectivity or bioavailability.

## Claims

1. A powder formulation for transpulmonary administration, inhalation or transnasal administration, which comprises:
(a) a mixture of fine particles containing a powdery medicament and erythritol as an excipient and having an average particle diameter of not more than 20 µm; and
(b) a carrier having a particle diameter of 10-200 µm wherein the carrier is erythritol.

2. The powder formulation according to claim 1, wherein the medicament is capable of self-aggregating through intermolecular interactions.

3. The powder formulation according to claim 1, wherein the medicament has a hygroscopic and/or deliquescent properties.

4. The powder formulation according to any one of claims 1 to 3,
wherein the medicament is a peptide or a protein.

5. The powder formulation according to any one of claims 1 to 4,
wherein the medicament is encapsulated within a lipid layer.

6. The powder formulation according to any one of claims 1 to 5,
wherein the proportion of powdery medicament to excipient is in the range of 1:5000 to 10:1by weight.

7. The powder formulation according to claims 1 to 6, wherein the proportion of fine particles to carrier is in the range of 1:100 to 10:1 1 by weight.

8. A method for producing a powder formulation as defined in claims 1 to 7, comprising the steps of:
dissolving or suspending a powdery medicament in a solution of erythritol as an excipient; spray-drying the resultant solution or suspension as such or disrupting a dried product obtained by lyophilizing the solution or suspension; thereby yielding fine particles having an average particle diameter of not more than 20 µm, and subsequently mixing the fine particles with a carrier having a particle diameter of 10-200 µm wherein the carrier is erythritol.

9. The method according to claim 8, wherein the medicament is capable of self-aggregating through intermolecular interactions.

10. A method for producing a powder formulation as defined in claims 1 to 7, comprising the steps of:
simultaneously mixing and grinding erythritol as an excipient and a powdery medicament using an aerodynamic grinder; thereby yielding fine particles having an average particle diameter of not more than 20 µm, and subsequently mixing the fine particles with a carrier having a particle diameter of 10-200 µm wherein the carrier is erythritol.

11. The method according to claim 10, wherein the medicament is capable of self-aggregating through intermolecular interactions.

12. An inhaler comprising a powder formulation as defined in any one of claims 1 to 7.

13. The powder formulation according to any one of claims 1 to 7,
wherein the fine particles can be easily separated from the carriers after administration and can reach target sites such as the bronchi or air vesicles.

## Patentansprüche

1. Pulverformulierung für die transpulmonale Verabreichung, Inhalation oder transnasale Verabreichung, welche umfaßt:
(a) eine Mischung aus feinen Partikeln, die ein pulverförmiges Medikament und Erythritol als Exzipienten enthalten und einen durchschnittlichen Partikeldurchmesser von nicht mehr als 20 µm haben; und
(b) einen Träger mit einem Partikeldurchmesser von 10-200 µm, wobei der Träger Erythritol ist.

2. Pulverformulierung gemäß Anspruch 1, wobei das Medikament in der Lage ist, durch intermolekulare Interaktionen selbst zu aggregieren.

3. Pulverformulierung gemäß Anspruch 1, wobei das Medikament hydroskopische und/oder zergehende Eigenschaften hat.

4. Pulverformulierung gemäß irgendeinem der Ansprüche 1 bis 3, wobei das Medikament in Peptid oder ein Protein ist.

5. Pulverformulierung gemäß irgendeinem der Ansprüche 1 bis 4, wobei das Medikament mit einer Lipidschicht verkapselt ist.

6. Pulverformulierung gemäß irgendeinem der Ansprüche 1 bis 5, wobei der Anteil des pulverförmigen Medikaments zum Exzipienten im Bereich von 1:5000 bis 10:1 nach Gewicht liegt.

7. Pulverformulierung gemäß Anspruch 1 bis 6, wobei der Anteil der feinen Partikel zum Träger im Bereich von 1:100 bis 10:1 nach Gewicht liegt.

8. Verfahren zum Herstellen einer Pulverformulierung wie in Ansprüchen 1 bis 7 definiert, umfassend die Schritte:
Lösen oder Suspendieren einer pulverförmigen Medikaments in einer Lösung aus Erythritol als Exzipienten;
Sprühtrocknen der erhaltenen Lösung oder Suspension als solche oder Aufschließen eines getrockneten Produkts, das durch Lyophilisieren der Lösung oder Suspension gewonnen wurde;
**dadurch** Gewinnen feiner Partikel mit einem durchschnittlichen Partikeldurchmesser von nicht mehr als 20 µm und
anschließendes Mischen der feinen Partikel mit einem Träger mit einem Partikeldurchmesser von 10-200 µm, wobei der Träger Erythritol ist.

9. Verfahren gemäß Anspruch 8, wobei das Medikament in der Lage ist, durch intermolekulare Interaktion selbst zu aggregieren.

10. Verfahren zum Herstellen einer Pulverformulierung wie in Ansprüchen 1 bis 7 definiert, umfassend die Schritte:
gleichzeitiges Mischen und Mahlen von Erythritol als Exzipienten und eines pulverförmigen Medikaments unter Verwendung eines aerodynamischen Mahlwerks;
**dadurch** Gewinnen feiner Partikel mit einem durchschnittlichen Partikeldurchmesser von nicht mehr als 20 µm und
anschließendes Mischen der feinen Partikel mit einem Träger mit einem Partikeldurchmesser von 10-200 µm, wobei der Träger Erythritol ist.

11. Verfahren gemäß Anspruch 10, wobei das Medikament in der Lage ist, durch intermolekulare Interaktion selbst zu aggregieren.

12. Inhalationsgerät umfassend einer Pulverformulierung wie in irgendeinem der Ansprüche 1 bis 7 definiert.

13. Pulverformulierung gemäß irgendeinem der Ansprüche 1 bis 7, wobei die feinen Partikel leicht von den Trägerstoffen nach Verabreichung getrennt werden können und die Zielorte, wie Bronchien oder Luftvesikel, erreichen können.

## Revendications

1. Formulation pulvérulente pour une administration transpulmonaire, une administration par inhalation ou transnasale, qui comprend :
(a) un mélange de fines particules contenant un médicament pulvérulent et de l'érythritol en tant qu'excipient et ayant un diamètre moyen de particule non supérieur à 20 µm; et
(b) un véhicule ayant un diamètre de particule de 10 à 200 µm, où le véhicule est l'érythritol.

2. Formulation pulvérulente selon la revendication 1, dans laquelle le médicament est capable de s'auto-agréger par des interactions intermoléculaires.

3. Formulation pulvérulente selon la revendication 1, dans laquelle le médicament présente des propriétés hygroscopiques et/ou déliquescentes.

4. Formulation pulvérulente selon l'une quelconque des revendications 1 à 3, dans laquelle le médicament est un peptide ou une protéine.

5. Formulation pulvérulente selon l'une quelconque des revendications 1 à 4, dans laquelle le médicament est encapsulé dans une couche lipidique.

6. Formulation pulvérulente selon l'une quelconque des revendications 1 à 5, dans laquelle la proportion de médicament pulvérulent par rapport à l'excipient se trouve dans la gamme de 1 : 5 000 à 10 : 1 en poids.

7. Formulation pulvérulente selon les revendications 1 à 6, dans laquelle la proportion de fines particules par rapport au véhicule se trouve dans la gamme de 1 : 100 à 10 : 1 en poids.

8. Procédé de production d'une formulation pulvérulente selon les revendications 1 à 7, comprenant les étapes consistant à :
dissoudre ou mettre en suspension un médicament pulvérulent dans une solution d'érythritol en tant qu'excipient ; sécher par pulvérisation la solution ou suspension résultante telle quelle ou rompre un produit séché obtenu en lyophilisant la solution ou suspension ; obtenir ainsi de fines particules ayant un diamètre moyen de particule non supérieur à 20 µm et mélanger ultérieurement les fines particules avec un véhicule ayant un diamètre de particule de 10 à 200 µm, où le véhicule est l'érythritol.

9. Procédé selon la revendication 8, dans lequel le médicament est capable de s'auto-agréger par des interactions intermoléculaires.

10. Procédé de production d'une formulation pulvérulente selon les revendications 1 à 7, comprenant les étapes consistant à :
mélanger et broyer simultanément l'érythritol en tant qu'excipient et un médicament pulvérulent en utilisant un broyeur aérodynamique ; donner ainsi de fines particules ayant un diamètre moyen de particule non supérieur à 20 µm et mélanger ultérieurement les fines particules avec un véhicule ayant un diamètre de particule de 10 à 200 µm, où le véhicule est l'érythritol.

11. Procédé selon la revendication 10, dans lequel le médicament est capable de s'auto-agréger par des interactions intermoléculaires.

12. Inhalateur comprenant une formulation pulvérulente selon l'une quelconque des revendications 1 à 7.

13. Formulation pulvérulente selon l'une quelconque des revendications 1 à 7, dans laquelle les fines particules peuvent être facilement séparées des véhicules après adtninistration et peuvent atteindre des sites cibles tels que les bronches ou les vésicules des voies aériennes.


### SEQUENCE LISTING

<110> ITOHAM FOODS INC.
<120> A Powder Inhalant and a Method for Producing the Same
<130> PH-1465
<160> 1
<170> Patent In Ver. 2.0
<210> 1
   <211> 31
   <212> PRT
   <213> Artificial Sequence
<220>
   <223> Description of Artificial Sequence: VIP derivative
<400> 1

His Ser Asp Ala Val Phe Thr Asp Asn Tyr Thr Arg Leu Arg Arg Gln
1               5                   10                  15
Met Ala Val Arg Arg Tyr Leu Asn Ser Ile Leu Asn Gly Arg Arg
20 25

### SEQUENCE LISTING

<110> ITOHAM FOODS INC.
   <120> Powdery Preparations and Process for Producing the Same
   <130> N.88926 GCW
<140> EP 01998330.3
   <141> 2001-11-29
<150> JP 2000-362704
   <151> 2000-11-29
<150> JP 2001-88337
   <151> 2001-03-26
<150> JP 2001-364325
   <151> 2001-11-29
<160> 1
   <170> Patent In Ver. 2.0
<210> 1
   <211> 31
   <212> PRT
   <213> Artificial Sequence
<220>
   <223> Description of Artificial Sequence: VIP derivative
<400> 1

His Ser Asp Ala Val Phe Thr Asp Asn Tyr Thr Arg Leu Arg Arg Gln
1               5                   10                  15
Met Ala Val Arg Arg Tyr Leu Asn Ser Ile Leu Asn Gly Arg Arg
20 25

### SEQUENCE LISTING

<110> ITOHAM FOODS INC.
<120> A Powder Inhalant and a Method for Producing the Same
<130> PH-1465
<160> 1
<170> Patent In Ver. 2.0
<210> 1
   <211> 31
   <212> PRT
   <213> Artificial Sequence
<220>
   <223> Description of Artificial Sequence: VIP derivative
<400> 1

His Ser Asp Ala Val Phe Thr Asp Asn Tyr Thr Arg Leu Arg Arg Gln
1               5                   10                  15
Met Ala Val Arg Arg Tyr Leu Asn Ser Ile Leu Asn Gly Arg Arg
            20                  25

### SEQUENCE LISTING

<110> ITOHAM FOODS INC.
   <120> Powdery Preparations and Process for Producing the Same
   <130> N.88926 GCW
<140> EP 01998330.3
   <141> 2001-11-29
<150> JP 2000-362704
   <151> 2000-11-29
<150> JP 2001-88337
   <151> 2001-03-26
<150> JP 2001-364325
   <151> 2001-11-29
<160> 1
   <170> Patent In Ver. 2.0
<210> 1
   <211> 31
   <212> PRT
   <213> Artificial Sequence
<220>
   <223> Description of Artificial Sequence: VIP derivative
<400> 1

His Ser Asp Ala Val Phe Thr Asp Asn Tyr Thr Arg Leu Arg Arg Gln
1               5                   10                  15
Met Ala Val Arg Arg Tyr Leu Asn Ser Ile Leu Asn Gly Arg Arg
            20                  25